# EUROPEAN PATENT SPECIFICATION

(11) **EP 2 539 261 B1**
(45) Date of publication and mention of the grant of the patent: **08.04.2020**
(21) Application number: 11707484.9
(22) Date of filing: 08.02.2011
(51) Int. Cl.: B65H 45/08

(54) **DEVICE AND PROCESS FOR FOLDING WEB MATERIALS**
VORRICHTUNG UND VERFAHREN ZUR FALTUNG VON BAHNMATERIALIEN
DISPOSITIF ET PROCÉDÉ POUR LE PLIAGE DE MATÉRIAUX EN BANDE

(30) Priority: 26.02.2010 IT TO20100143
(43) Date of publication of application: 02.01.2013
(73) Proprietor: Fameccanica.Data S.p.A., 65129 Pescara (IT)
(72) Inventor: SABLONE, Gabriele, 66015 Montesilvano (Pescara) (IT); PASQUALONI, Paolo, 66020 Sambuceto di San Giovanni Teatino (Chieti) (IT)
(74) Representative: Bosotti, Luciano
(86) International application number: PCT/IB2011/050527
(87) International publication number: WO 2011/104647

(56) References cited:
- EP-A1- 0 437 848
- WO-A1-2012/088062
- WO-A2-02/068301

## Description

### Field of the invention

The present disclosure relates to the techniques for folding web materials.

The disclosure has been developed with particular attention paid to its possible application to the folding of web materials used in the production of sanitary articles.

### Description of the prior art

In numerous sectors of the art there arises the need to fold web materials moving in the longitudinal direction of the web. For instance, in the packaging industry machines are known referred to as "flow-pack" (or "ffs", acronym for "form-fill-seal"), where a film winding material gives rise to a closed tubular semifinished product obtained by sealing together, so as to form a so-called longitudinal "fin", the edges of the web material after it has been closed on itself in a device commonly referred to as "former". An example of such a former is described in the document No. US-A-4 761 937. In the production of sanitary articles it is altogether common practice to use folding devices substantially constituted by a sort of plough share over which a web that is advancing is run and folded in a V-shape, without any interruption of its movement of advance. Such a device is illustrated, for instance, in the document No. US-A-3 066 932. More specifically, the invention relates to a device according to the preamble of claim 1, which is known e.g. from WO 02/068301 A2. Also EP 0 437 848 A1 is of some interest for the invention.

### Object and summary of the invention

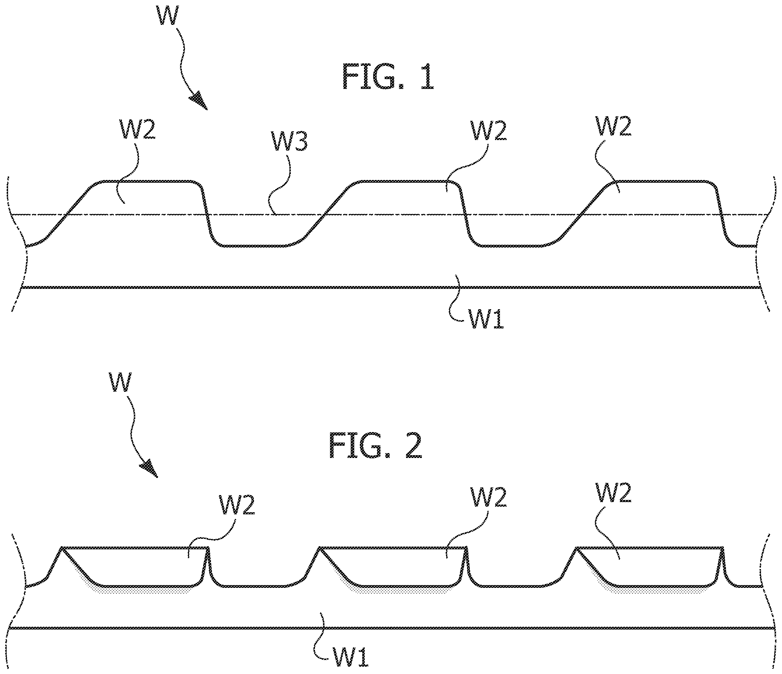

The inventors have noted that folding devices of a conventional type end up being mostly unusable in the case where the web material is, for instance, a web material W of the type schematically illustrated in Figures 1 and 2, i.e., a material having a profile that varies according to a general configuration that can be defined as "sawtooth" and hence comprising a continuous core part W1 from which a regular sequence of "teeth" W2 extends, where the material W is to be by folded to form a V according to a line W3 that extends approximately half way up the "teeth" W2.

A situation of the type illustrated may be found, for example, during execution of operations of folding of so-called side panels of sanitary articles that can be worn as a pair of pants. Making the fold of a material W, as schematically illustrated in Figures 1 and 2, with a conventional folding or shaping device (of a folding-blade type or the like) proves practically impossible: the aforesaid folding blade (or equivalent folding element) tends in fact to jam in the voids between successive teeth W2.

To these considerations it should also be added the fact that, in particular in the production of sanitary articles, webs like the web W illustrated in Figures 1 and 2 can be made of rather thin material, with the further difficulty represented by the fact that, after folding, the crest parts of the teeth W2 tend inevitably to "flutter" in an undesirable way.

Moreover, in the case where extremely thin and hence rather delicate web materials are used (consider, for example, films of nonwoven fabric of extremely low substance, which find increasing use in the sector of sanitary articles), the folding operation can become critical even when the web material has a regular and constant profile, presenting as a normal web. In this case, the interaction of the folding blade or other active element of the folding device with a very delicate material runs the risk of leading to undesirable damage of the material itself, also taking into account the fact that in sectors like the ones considered involve extremely high automatic production rates, indeed, increasingly high production rates, to which there corresponds a constant increase in the speed of advance of the webs that are to undergo the folding operation.

The need to have available improved folding solutions is hence felt not only in relation to web materials with an irregular profile, for example, a sawtooth profile, as illustrated in Figures 1 and 2, but also in the case of web materials with a regular, constant, profile constituted by materials that are rather thin and delicate, which hence find it hard to tolerate any excessively violent operation of manipulation.

The object of the present invention is to provide an answer to said need.

According to the invention, said object is achieved thanks to a device having the characteristics recalled specifically in claim 1. The invention also regards a corresponding process as called for in claim 7. The claims form an integral part of the technical teaching provided herein in relation to the invention.

### Brief description of the annexed drawings

The invention will now be described, purely by way of non-limiting example, with reference to the annexed drawings, in which:
- Figures 1 and 2 have already been described previously;
- Figures 3 to 5 illustrate, in progressively greater depth, the principles of operation of embodiments of the invention; and
- Figures 6, 7, and 8 are successive cross-sectional views according to the lines VI-VI, VII-VII and VIII-VIII of Figure 5.

### Detailed description of embodiments

Illustrated in the ensuing description are various specific details aimed at an in-depth understanding of the embodiments. The embodiments can be produced without one or more of the specific details, or with other methods, components, materials, etc. In other cases, known structures, materials, or operations are not illustrated or described in detail herein so as not to render various aspects of the embodiments obscure.

The reference to "an embodiment" or "one embodiment" in the framework of the present description is intended to indicate that a particular configuration, structure, or characteristic described in relation to the embodiment is comprised in at least one embodiment. Hence, phrases such as "in an embodiment" or "in one embodiment" that may be present in different points of the present description do not necessarily refer to one and the same embodiment. Furthermore, particular conformations, structures, or characteristics can be adequately combined in one or more embodiments.

The references used herein are provided merely for convenience and hence do not define the sphere of protection or the scope of the embodiments.

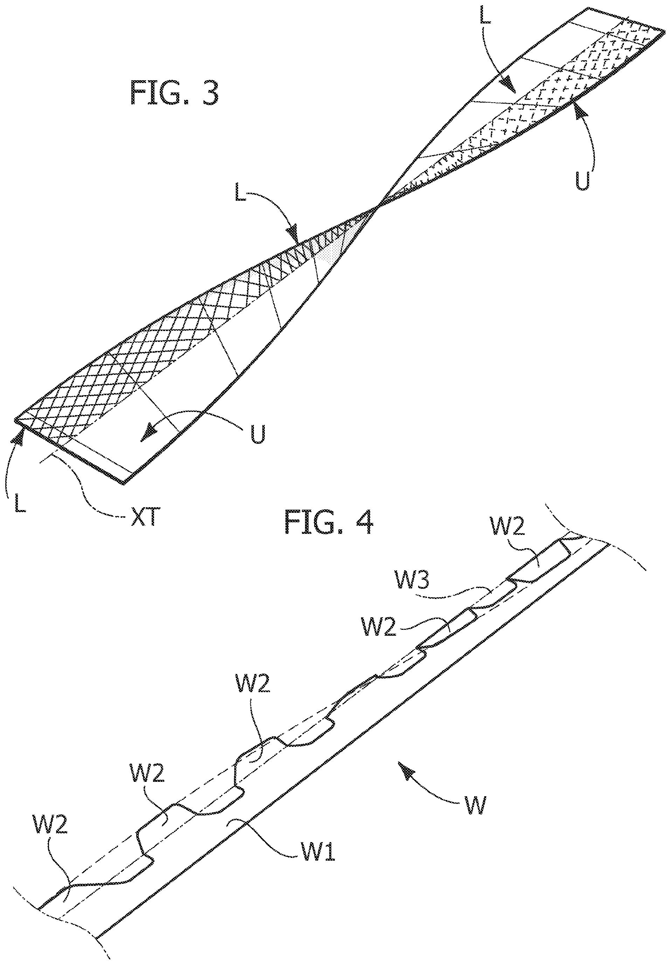

The drawing of Figure 3 is a schematic illustration of the behaviour of a rectangular lamina T (in practice, a length of belt) when said lamina is subjected to helical twisting about a median axis XT thereof.

In particular, when a torsion is imparted on the lamina T such as to bring its opposite ends to be rotated with respect to each other through 180° about the axis XT:
- what at one end of the lamina T (for instance, at the end in the foreground in Figure 3) is the top surface U, at the opposite end of the lamina T, is facing downwards; and
- what at one end of the lamina T (consider once again the end in the foreground in Figure 3) is the "bottom" surface L, at the opposite end of the lamina T, is facing upwards.

From an observation of half of the (initially top) face U, which is hatched in the part in the foreground in Figure 3, it may be noted that moving gradually towards the other end of the lamina T, said half-surface follows a helical movement about the axis XT in such a way that the aforesaid half, initially lying in a horizontal plane, starts gradually to turn with respect to the axis XT (in a clockwise direction, as viewed in Figure 3) to assume an approximately vertical position half-way along the lamina T and then face downwards, as schematically indicated by the part hatched with dashed lines in the background of Figure 3.

Various embodiments are based upon the recognition of the fact that, with reference by way of non-limiting example to the web W of Figures 1 and 2, the desired movement of folding of the crest parts of the teeth W2 about the axis W3 can be obtained:
- by keeping the core part W1 in a condition of advance (for example, of sliding) on a horizontal plane; and
- bringing the crest parts of the teeth W2 to advance (and in particular to be conveyed) by the moving portion of a belt corresponding to the dashed part of the lamina T of Figure 3.

In this way (as schematically represented in Figure 4), the crest parts of the teeth W2, which are initially set in a horizontal plane, co-planar and projecting from the core part W1, are gradually turned over upwards into a vertical position and then are definitively folded to form a V up against the core part W1.

The folding function represented schematically in Figure 4 can be obtained also on a web material that has a profile altogether different, including a normal profile with constant section of a normal web, ensuring a regular advance of the material W also when this presents a markedly irregular profile (see, for example, Figures 1 and 2) and/or is particularly delicate and sensitive (being constituted for instance, by a very thin nonwoven fabric, of low substance).

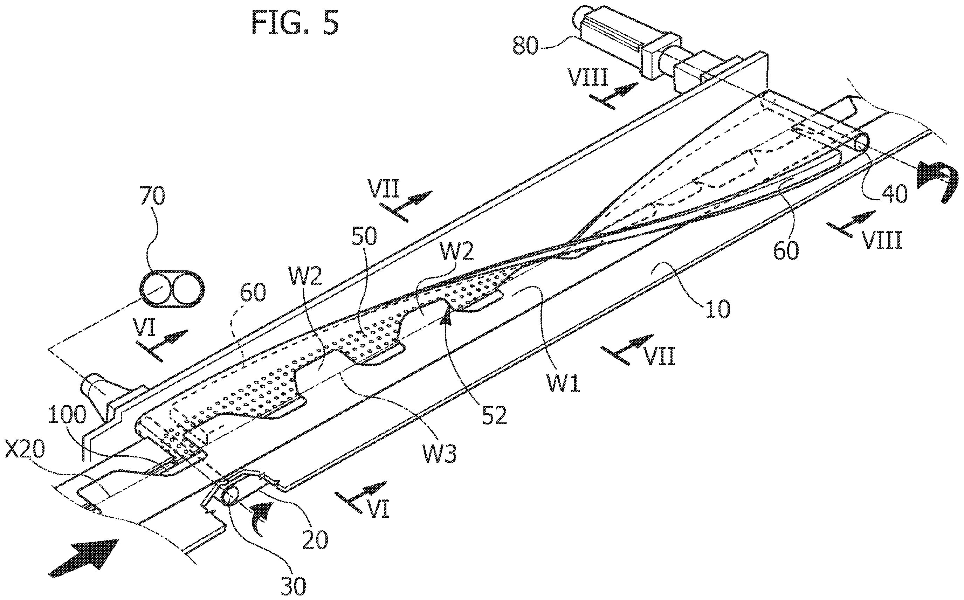

Figure 5 shows a possible embodiment of the folding mechanism represented ideally by the sequence of Figures 3 and 4.

In particular, the reference 10 designates a plate that is to constitute a surface of sliding (from left to right, as viewed in Figure 5) for the core part W1 of the web W. The plate 10 has a side edge 100 that is to identify, as will be more clearly seen in what follows, the line W3 at which the action of folding of the web W is exerted.

The reference number 20 designates, instead, a belt conveyor set alongside the plate 10 so as to be practically coextensive with the edge 100. The conveyor 20 can be of the type commonly used in the automatic-packaging industry and also in the sector of the production of sanitary articles. It may, for instance, be a strong belt made of flexible plastic material (possibly provided with a textile reinforcement: the corresponding technological solutions are extremely numerous) forming an endless loop by running over two mutually parallel end rollers 30 and 40, where at least one of them (for example, the roller 40) is assumed as being moved by a power drive 80, of a known type.

When the power drive 80 is active, the top branch of the belt travels upwards along its path running over the roller 30 (which is assumed as here turning in a clockwise direction) and then advances towards the roller 40.

The condition of mounting of the rollers 30 and 40 is such that, in the passage from the roller 30 to the roller 40 situated at the opposite end of the conveyor, the belt 20 is subject to a helical movement of torsion through 180° around a median axis X20 thereof, substantially aligned with the edge 100 of the plate 10.

In this way, what in the proximity of the roller 30 is the top branch of the belt 20, which is to receive and draw along on it the toothed parts W2 of the web W - which are currently co-planar with the core part W1 -, at the opposite end of the conveyor (in the background of Figure 5) comes to be facing downwards, so as to follow upwards its path of running over the roller 40 (which is here assumed to be turning in a counterclockwise direction) and then once again advances towards the roller 30.

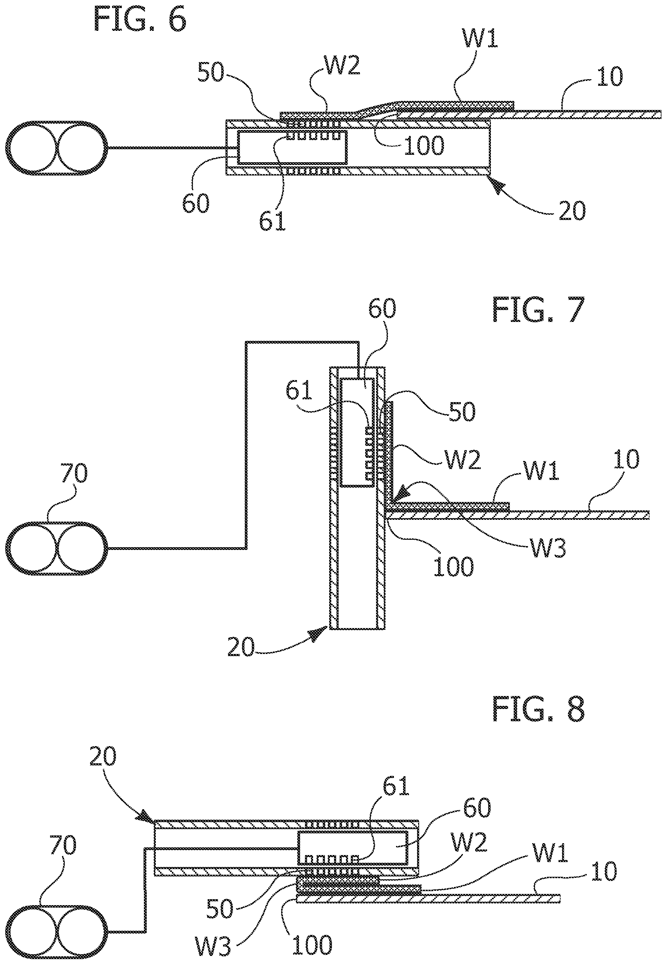

The observation in sequence of the three cross-sectional views of Figures 6, 7, and 8 makes it possible to realize the effect of the helical twisting path imparted on the belt conveyor 20.

In particular, if we assume feeding to the belt conveyor 20 starting from its end set upstream (on the left in Figure 5, where the web W is assumed to be advancing from left to right) the web material W to be subjected to folding, it is possible to cause initially - as illustrated in Figure 6 - the core part W1 of the web W to slide along the plate 10 whilst the crest parts of the teeth W2 are drawn by the top branch of the conveyor 20, which is practically coplanar with (in actual fact set slightly below) the plate 10.

Once approximately half-way of the longitudinal development of the conveyor 20 is reached (see the cross-sectional view of Figure 7), as a result of the gradual helical twisting - to the right or in a clockwise direction, in the example illustrated herein - about the axis X20, the part of the top branch of the conveyor belt 20 on which the crest parts of the teeth W2 rest reaches a vertical position and imparts a corresponding movement on the crest parts of the teeth W2, which are thus folded at approximately 90° with respect to the core part W1 along the line W3 (side 100 of the plate 10).

Once the belt conveyor 20 arrives in the proximity of the roller 40 (see cross-sectional view of Figure 8), it is completely turned over above the plate 10, so that what was originally the top conveying branch of the belt conveyor 20 has become the bottom branch, which moves over the plate 10 at a slight distance therefrom, the belt conveyor 20 maintaining (and drawing along with it) the web W with the crest parts of the teeth W2 folded to form a V up against the core part W1 along the line W3 (side 100 of the plate 10).

The device illustrated makes it possible to form, in a web element W that is advancing, a fold along a line of fold W3 oriented in the direction of advance of the belt, which brings a first portion (e.g., W1) and a second portion (e.g., W2) of the web element W, which are initially situated on opposite sides of the line of fold W3 (see Figure 1), to assume a folded configuration of the type represented in Figure 2, in which the second portion W2 is turned over on the first portion W1.

The person skilled in the sector will appreciate that to provide the surface of feed 10 it is possible to resort to solutions different from a sliding plate; for instance, in various embodiments, the surface of feed 10 can be defined by one of the two branches of a belt conveyor of the type commonly used in the automatic-packaging industry and also in the sector of production of sanitary articles.

The reference 50 designates an array of holes, which may be provided, according to criteria in themselves known, in the portion of the conveyor belt 20 that is to co-operate with the crest parts W2 subjected to folding.

In various embodiments, the array o holes 50 may concern only the area on which the crest parts W2 of the web come to rest.

The array of holes or openings 50 is to co-operate with a suction box 60 set within the belt conveyor 20 in a condition of intermediate extension between the two branches of delivery and return of the conveyor itself. The suction box 60 is provided with suction mouths 61 and can be set in communication with a source of subatmospheric pressure 70 (a so-called "vacuum pump"). The source 70 has the function of inducing a flow of aeriform from the external environment towards the inside of the suction box 60, said flow passing through the openings 50 of the belt and the suction mouths 61 from the suction box 60.

According to a solution known in the sector of automatic packaging and in the sector of manufacture of sanitary products, the so-called "vacuum cleaner effect" thus induced means that the air recalled from the surrounding environment into the suction box 60 performs an action of recall such as to withhold against the conveyor belt 20 the crest parts W2 of the web material W preventing detachment thereof from the conveyor belt and their undesirable "fluttering" during execution of the folding operation.

Said effect of withholding and providing a sure guide in the course of execution of the folding operation can be achieved also in the case of a web material having a regular profile (hence with constant section) made, however, of a particularly delicate material, for instance, a nonwoven fabric with particularly low substance.

Figure 5 and the complex of the cross-sectional views of Figures 6 to 8 exemplify the fact that in various embodiments the suction box 60 has a helical configuration twisted about an axis substantially corresponding to the axis X20. Said configuration does not hinder regular operation either of the conveyor 20 or of the suction box 60.

In various embodiments, the suction box 60 can comprise a single suction chamber. In various embodiments, the suction box can comprise distinct chambers, each connected to a respective line for application of a subatmospheric pressure (vacuum line).

As may be inferred from Figures 6 to 8, the suction box 60 can occupy only approximately half of the development of the internal space of the belt conveyor 20, where the array of holes 50 is present. Likewise, the transverse dimensions of said belt conveyor could be smaller than in the example of embodiment illustrated, for example, envisaging that the conveyor belt has a width only a little larger than the width of the suction box 60, which is set sandwiched between the two branches of the belt.

In various embodiments, the belt 20 has, however, a greater extension, as represented in the drawings. In various embodiments, this enables the action of grip of the belt on the web material W subjected to folding to be rendered more regular and continuous, preventing any possible of uncovered areas from accidentally arising.

In various embodiments, the structure described herein is suited also to being produced in a paired or "twin" form using two devices of the type illustrated in Figures 5 and 8 set alongside one another and specularly symmetrical so as to produce simultaneously the V-shaped fold of two webs W that advance parallel to one another. The two devices thus paired can possibly share the plate 10, which in this case will have two opposite parallel sides, each facing a respective belt conveyor, with the two conveyors presenting helical twisting paths that are opposite and mutually convergent (one in a clockwise direction, the other in a counterclockwise direction). In some applications, the two webs W that advance parallel to one another can even be united in just one web, upon which a C-shaped folding profile can be imparted by folding the two opposite edges of the web itself to form a V.

Of course, without prejudice to the principle of the invention, the details of production and the embodiments may vary, even significantly, with respect to what is illustrated herein purely by way of non-limiting example, without thereby departing from the scope of the invention as defined by the annexed claims.

## Claims

1. A device for folding an advancing web element (W) along a folding line (W3) extending in the direction of advancement of the web so as to bring a first (W1) and a second (W2) portion of said web element (W) arranged on opposite sides of the folding line (W3) into a folded configuration wherein said second portion (W) is folded over onto said first portion (W1), the device including:
- an advancement plane (10) for said first portion (W1) of said web element (W), said plane having a folding edge (100) to define said folding line (W3),
- a motorized belt conveyor (20) arranged sidewise said advancement plane (10) along said folding edge (100) to transport said second portion (W2) of said web element (W) undergoing folding; said belt conveyor (20) following a helix-like screwing trajectory around said folding edge (100), said screwing trajectory brings the upper branch of said belt conveyor (20), which is initially co-planar with said advancement plane (10), to turn over onto and superpose to said advancement plane (10) to effect said folding of said web element (W), **characterized in that**:
- said belt conveyor (20) has an apertured strip (50) to receive resting thereon said second portion (W2) of said web element (W) undergoing folding, and
- within said belt conveyor (20) there is provided an aspiration box (60) to produce an air flow into said belt conveyor (20) through said apertured strip (50) so as to draw said second portion (W2) of the web element (W) into contact with the belt conveyor (20).

2. The device of claim 1, wherein said belt conveyor (20) includes two counter-rotating end rollers (30, 40) arranged on opposite sides with respect to said advancement plane (100).

3. The device of claim 2, wherein said belt conveyor (20) has an input end for receiving said web element (W) to be folded and an output end to output said web element (W) once folded, wherein said two end rollers (30, 40) are arranged below said advancement plane (10) at said input end and above said advancement plane (10) at said output end, respectively.

4. The device of claim 2 or claim 3, wherein said end rollers (30, 40) are parallel to each other.

5. The device of any of the previous claims, wherein said advancement plane (10) includes one of the branches of a belt conveyor.

6. An arrangement including the combination of two devices of any of the previous claims, the two devices set alongside one another and specularly symmetrical so as to produce simultaneously the V-shaped fold of two web elements (W) that advance parallel to one another, the two symmetric devices including respective belt conveyors having opposed screwing directions, with the two conveyors presenting helical twisting paths that are opposite and mutually convergent.

7. A method of folding an advancing web element (W) along a folding line (W3) extending in the direction of advancement of the web so as to bring a first (W1) and a second (W2) portion of said web element (W) arranged on opposite sides of the folding line (W3) into a folded configuration wherein said second portion (W) is folded over onto said first portion (W1), the method including:
- advancing along an advancement plane (10) said first portion (W1) of said web element (W) undergoing folding, said plane having a folding edge (100) to define said folding line (W3),
- arranging sidewise said advancement plane (10) along said folding edge (100) a motorized belt conveyor (20) to transport said second portion (W2) of said web element (W) undergoing folding, said belt conveyor (20) following a helix-like screwing trajectory around said folding edge (100), said screwing trajectory brings the upper branch of said belt conveyor (20), which is initially co-planar with said advancement plane (10), to turn over onto and superpose to said advancement plane (10) to effect said folding of said web element (W), the method **characterized in that** it includes:
- drawing said second portion (W2) of the web element (W) into contact with the belt conveyor (20), said belt conveyor (20) having an apertured strip (50) to receive resting thereon said second portion (W2) of said web element (W) undergoing folding, within said belt conveyor (20) there being provided an aspiration box (60) to produce an air flow into said belt conveyor (20) through said apertured strip (50).

## Patentansprüche

1. Einrichtung zum Falten eines vorlaufenden Bahnelements (W) entlang einer sich in der Vorlaufrichtung der Bahn erstreckenden Faltlinie (W3), um einen ersten (W1) und einen zweiten (W2) Abschnitt des Bahnelements (W), welche auf gegenüberliegenden Seiten der Faltlinie (W3) angeordnet sind, in eine gefaltete Anordnung zu bringen, in welcher der zweite Abschnitt (W) auf den ersten Abschnitt (W1) umgefaltet ist, wobei die Einrichtung Folgendes beinhaltet:
- eine Vorlaufebene (10) für den ersten Abschnitt (W1) des Bahnelements (W), wobei die Ebene eine Faltkante (100) aufweist, um die Faltlinie (W3) zu definieren,
- einen motorgetriebenen Bandförderer (20), der seitlich zu der Vorlaufebene (10) entlang der Faltkante (100) angeordnet ist, um den zweiten Abschnitt (W2) des die Faltung durchlaufenden Bahnelements (W) zu transportieren, wobei der Bandförderer (20) einer spiralartigen Schraubenbahn um die Faltkante (100) herum folgt, wobei die Schraubenbahn den oberen Teil des Bandförderers (20), welcher anfangs koplanar mit der Vorlaufebene (10) ist, dazu bringt, sich auf die Vorlaufebene (10) umzuwenden und diese zu überlagern, um die Faltung des Bahnelements (W) zu bewirken, **dadurch gekennzeichnet, dass**:
- der Bandförderer (20) einen mit Öffnungen versehenen Streifen (50) aufweist, um auf diesem aufliegend den zweiten Abschnitt (W2) des die Faltung durchlaufenden Bahnelements (W) aufzunehmen, und
- innerhalb des Bandförderers (20) ein Saugkasten (60) bereitgestellt ist, um einen durch den mit Öffnungen versehenen Streifen (50) hindurch in den Bandförderer (20) hinein verlaufenden Luftstrom zu erzeugen, um den zweiten Abschnitt (W2) des Bahnelements (W) in Kontakt mit dem Bandförderer (20) zu ziehen.

2. Einrichtung nach Anspruch 1, wobei der Bandförderer (20) zwei gegenläufig drehende Endwalzen (30, 40) beinhaltet, die in Bezug auf die Vorlaufebene (100) auf entgegengesetzten Seiten angeordnet sind.

3. Einrichtung nach Anspruch 2, wobei der Bandförderer (20) ein Eingangsende zum Aufnehmen des zu faltenden Bahnelements (W) und ein Ausgabeende zum Ausgeben des gefalteten Bahnelements (W) aufweist, wobei die beiden Endwalzen (30, 40) an dem Eingangsende unterhalb der Vorlaufebene (10) bzw. an dem Ausgabeende oberhalb der Vorlaufebene (10) angeordnet sind.

4. Einrichtung nach Anspruch 2 oder Anspruch 3, wobei die Endwalzen (30, 40) parallel zueinander liegen.

5. Einrichtung nach einem der vorhergehenden Ansprüche, wobei die Vorlaufebene (10) einen der Abschnitte eines Bandförderers beinhaltet.

6. Anordnung, welche die Kombination aus zwei Einrichtungen nach einem der vorhergehenden Ansprüche beinhaltet, wobei die beiden Einrichtungen längsseits nebeneinandergesetzt und spiegelsymmetrisch sind, um gleichzeitig die V-förmige Faltung zweier Bahnelemente (W) zu erzeugen, die parallel zueinander vorlaufen, wobei die beiden symmetrischen Einrichtungen jeweilige Bandförderer mit entgegengesetzten Schraubenrichtungen aufweisen, wobei die beiden Förderer sich spiralförmig windende Wege aufweisen, die entgegengesetzt und zueinander konvergent sind.

7. Verfahren zum Falten eines vorlaufenden Bahnelements (W) entlang einer sich in der Vorlaufrichtung der Bahn erstreckenden Faltlinie (W3), um einen ersten (W1) und einen zweiten (W2) Abschnitt des Bahnelements (W), welche sich auf gegenüberliegenden Seiten der Faltlinie (W3) befinden, in eine gefaltete Anordnung zu bringen, in welcher der zweite Abschnitt (W) auf den ersten Abschnitt (W1) umgefaltet ist, wobei das Verfahren Folgendes beinhaltet:
- Vortreiben des ersten Abschnitts (W1) des die Faltung durchlaufenden Bahnelements (W) entlang einer Vorlaufebene (10), wobei die Ebene eine Faltkante (100) aufweist, um die Faltlinie (W3) zu definieren,
- Anordnen eines motorgetriebenen Bandförderers (20) seitlich zu der Vorlaufebene (10) entlang der Faltkante (100), um den zweiten Abschnitt (W2) des die Faltung durchlaufenden Bahnelements (W) zu transportieren, wobei der Bandförderer (20) einer spiralartigen Schraubenbahn um die Faltkante (100) herum folgt, wobei die Schraubenbahn den oberen Abschnitt des Bandförderers (20), welcher anfangs koplanar mit der Vorlaufebene (10) ist, dazu bringt, sich auf die Vorlaufebene (10) umzuwenden und diese zu überlagern, um die Faltung des Bahnelements (W) zu bewirken, wobei das Verfahren **dadurch gekennzeichnet ist, dass** es Folgendes beinhaltet:
- Ziehen des zweiten Abschnitts (W2) des Bahnelements (W) in Kontakt mit dem Bandförderer (20), wobei der Bandförderer (20) einen mit Öffnungen versehenen Streifen (50) aufweist, um auf diesem aufliegend den zweiten Abschnitt (W2) des die Faltung durchlaufenden Bahnelements (W) aufzunehmen, wobei innerhalb des Bandförderers (20) ein Saugkasten (60) bereitgestellt ist, um einen durch den mit Öffnungen versehenen Streifen (50) hindurch in den Bandförderer (20) hinein verlaufenden Luftstrom zu erzeugen.

## Revendications

1. Dispositif pour plier un élément de bande en mouvement (W) le long d'une ligne de pliage (W3) s'étendant dans la direction d'avancement de la bande de manière à amener des première (W1) et deuxième (W2) parties dudit élément de bande (W) agencées sur des côtés opposés de la ligne de pliage (W3) dans une configuration pliée dans laquelle ladite deuxième partie (W2) est repliée sur ladite première partie (W1), le dispositif comportant :
- un plan d'avancement (10) pour ladite première partie (W1) dudit élément de bande (W), ledit plan ayant un bord de pliage (100) pour définir ladite ligne de pliage (W3),
- un transporteur à courroie motorisé (20) agencé latéralement audit plan d'avancement (10) le long dudit bord de pliage (100) pour transporter ladite deuxième partie (W2) dudit élément de bande (W) subissant un pliage ; ledit transporteur à courroie (20) suivant une trajectoire de vissage hélicoïdale autour dudit bord de pliage (100), ladite trajectoire de vissage amène la branche supérieure dudit transporteur à courroie (20), qui est initialement coplanaire avec ledit plan d'avancement (10), à se retourner sur et à se superposer audit plan d'avancement (10) pour effectuer ledit pliage dudit élément de bande (W), **caractérisé en ce que** :
- ledit transporteur à courroie (20) a un ruban à ouvertures (50) pour recevoir ladite deuxième partie (W2) dudit élément de bande (W) subissant un pliage en étant en appui sur celui-ci, et
- à l'intérieur dudit transporteur à courroie (20), une boîte d'aspiration (60) est prévue pour produire un flux d'air dans ledit transporteur à courroie (20) à travers ledit ruban à ouvertures (50) de manière à tirer ladite deuxième partie (W2) de l'élément de bande (W) pour entrer en contact avec le transporteur à courroie (20).

2. Dispositif de la revendication 1, dans lequel ledit transporteur à courroie (20) comporte deux rouleaux d'extrémité contrarotatifs (30, 40) agencés sur des côtés opposés par rapport audit plan d'avancement (100).

3. Dispositif de la revendication 2, dans lequel ledit transporteur à courroie (20) a une extrémité d'entrée pour recevoir ledit élément de bande (W) à plier et une extrémité de sortie pour faire sortir ledit élément de bande (W) une fois plié, dans lequel lesdits deux rouleaux d'extrémité (30, 40) sont agencés en dessous dudit plan d'avancement (10) à ladite extrémité d'entrée et au-dessus dudit plan d'avancement (10) à ladite extrémité de sortie, respectivement.

4. Dispositif de la revendication 2 ou 3, dans lequel lesdits rouleaux d'extrémité (30, 40) sont parallèles l'un à l'autre.

5. Dispositif de l'une des revendications précédentes, dans lequel ledit plan d'avancement (10) comporte l'une des branches d'un transporteur à courroie.

6. Agencement comportant la combinaison de deux dispositifs de l'une des revendications précédentes, les deux dispositifs étant placés l'un à côté de l'autre et spéculairement symétriques de manière à produire simultanément le pli en forme de V de deux éléments de bande (W) qui avancent parallèlement l'un à l'autre, les deux dispositifs symétriques comportant des transporteurs à courroie respectifs ayant des directions de vissage opposées, les deux transporteurs présentant des chemins de torsion hélicoïdaux qui sont opposés et mutuellement convergents.

7. Procédé de pliage d'un élément de bande en mouvement (W) le long d'une ligne de pliage (W3) s'étendant dans la direction d'avancement de la bande de manière à amener des première (W1) et deuxième (W2) parties dudit élément de bande (W) agencées sur des côtés opposés de la ligne de pliage (W3) dans une configuration pliée dans laquelle ladite deuxième partie (W2) est repliée sur ladite première partie (W1), le procédé comportant le fait :
- de faire avancer le long d'un plan d'avancement (10), ladite première partie (W1) dudit élément de bande (W) subissant un pliage, ledit plan ayant un bord de pliage (100) pour définir ladite ligne de pliage (W3),
- d'agencer ledit plan d'avancement (10) le long dudit bord de pliage (100) latéralement à un transporteur à courroie motorisé (20) pour transporter ladite deuxième partie (W2) dudit élément de bande (W) subissant un pliage, ledit transporteur à courroie (20) suivant une trajectoire de vissage hélicoïdale autour dudit bord de pliage (100), ladite trajectoire de vissage amène la branche supérieure dudit transporteur à courroie (20), qui est initialement coplanaire avec ledit plan d'avancement (10), à retourner sur et à se superposer audit plan d'avancement (10) pour effectuer ledit pliage dudit élément de bande (W), le procédé étant **caractérisé en ce qu'**il comporte le fait :
- de tirer ladite deuxième partie (W2) de l'élément de bande (W) pour entrer en contact avec le transporteur à courroie (20), ledit transporteur à courroie (20) ayant un ruban à ouvertures (50) pour recevoir ladite deuxième partie (W2) dudit élément de bande (W) subissant un pliage en étant en appui sur celui-ci, à l'intérieur dudit transporteur à courroie (20) une boîte d'aspiration (60) étant prévue pour produire un flux d'air dans ledit transporteur à courroie (20) à travers ledit ruban à ouvertures (50).
